# EUROPEAN PATENT SPECIFICATION

(11) **EP 2 121 011 B1**
(45) Date of publication and mention of the grant of the patent: **21.05.2014**
(21) Application number: 07866632.8
(22) Date of filing: 05.12.2007
(51) Int. Cl.: C12N 7/00, A61K 39/145

(54) **VACCINES INCLUDING ANTIGEN FROM FOUR STRAINS OF INFLUENZA VIRUS**
IMPFSTOFFE MIT ANTIGEN AUS VIER STÄMMEN DES INFLUENZAVIRUS
VACCINS COMPRENANT UN ANTIGÈNE ISSU DE QUATRE SOUCHES DU VIRUS GRIPPAL

(30) Priority: 06.12.2006 US 873815 P
(43) Date of publication of application: 25.11.2009
(60) Divisional application: 12180148.4
(73) Proprietor: Novartis AG, 4056 Basel (CH)
(72) Inventor: TSAI, Theodore F., Philadelphia, PA 19103 (US)
(74) Representative: Marshall, Cameron John
(86) International application number: PCT/IB2007/004364
(87) International publication number: WO 2008/068631

(56) References cited:
- WO-A-2006/100110
- WO-A2-2008/032219
- US-A1- 2005 186 621
- SCHNEIDER M M ET AL: "Antibody response to tetravalent influenza subunit vaccine in patients infected with human immunodeficiency virus type 1." INTERNATIONAL JOURNAL OF ANTIMICROBIAL AGENTS APR 1996, vol. 6, no. 4, April 1996 (1996-04), pages 195-200, XP002493637 ISSN: 0924-8579
- A. MEIJER ET AL: 'Euroroundup: EPIDEMIOLOGICAL AND VIROLOGICAL ASSESSMENT OF INFLUENZA ACTIVITY IN EUROPE, DURING THE 2004-2005 WINTER' 01 May 2006, pages 1 - 15, XP055052617
- GIOVANNI DELLA CIOPPA ET AL: "Trivalent and quadrivalent MF59-adjuvanted influenza vaccine in young children: A dose- and schedule-finding study", VACCINE, ELSEVIER LTD, GB, vol. 29, no. 47, 25 August 2011 (2011-08-25), pages 8696-8704, XP028325119, ISSN: 0264-410X, DOI: 10.1016/J.VACCINE.2011.08.111 [retrieved on 2011-08-31]
- 'MEDIMMUNE ANNOUNCES FDA APPROVAL OF FIRST FOUR-STRAIN FLU VACCINE, FLUMIST® QUADRIVALENT (INFLUENZA VACCINE LIVE, INTRANASAL)', [Online] 29 February 2012, Retrieved from the Internet: <URL:http://pressroom.medimmune.com/press-r eleases/2012/02/29/medimmune-announces-fda- approval-of-flumist%c2%ae-quadrivalent-infl uenza-vaccine-live-intranasal/>
- 'Three years follow up study of national influenza vaccination practices in Japan' JOURNAL OF EPIDEMIOLOGY AND COMMUNITY HEALTH, [Online] vol. 48, no. 1, 01 January 1994, pages 46 - 51, XP055052125 Retrieved from the Internet: <URL:http://jech.bmj.com/content/48/1/46.fu ll.pdf#page=1&view=FitH> [retrieved on 2013-02-01]
- GOVAERT TH M E ET AL: "Immune response to influenza vaccination of elderly people. A randomized double-blind placebo-controlled trial", VACCINE, ELSEVIER LTD, GB, vol. 12, no. 13, 1 January 1994 (1994-01-01), pages 1185-1189, XP023662823, ISSN: 0264-410X, DOI: 10.1016/0264-410X(94)90241-0 [retrieved on 1994-01-01]
- WOOD J M: "Selection of influenza vaccine strains and developing pandemic vaccines", VACCINE, ELSEVIER LTD, GB, vol. 20, 20 December 2002 (2002-12-20), pages B40-B44, XP004397486, ISSN: 0264-410X, DOI: 10.1016/S0264-410X(02)00509-1
- LEVANDOWSKI R A ET AL: "ANTIBODY RESPONSES TO INFLUENZA B VIRUSES IN IMMUNOLOGICALLY UNPRIMED CHILDREN", PEDIATRICS, AMERICAN ACADEMY OF PEDIATRICS, EVANSTON, IL, US, vol. 88, no. 5, 1 November 1991 (1991-11-01), pages 1031-1036, XP009166781, ISSN: 0031-4005
- H.-P. TSAI ET AL: "Increasing Appearance of Reassortant Influenza B Virus in Taiwan from 2002 to 2005", JOURNAL OF CLINICAL MICROBIOLOGY, vol. 44, no. 8, 1 August 2006 (2006-08-01) , pages 2705-2713, XP055052616, ISSN: 0095-1137, DOI: 10.1128/JCM.02694-05
- CHI-HO CHAN, YOU CHAN, HAPPY-K SHIEH, CHENG-HSIEN TSAI, CHIA-YUAN CHEN, SHU-CHIH LIU, WU-TSE LIU: 'Phylogenetic analysis of influenza B virus in Taiwan,1997 to 2001' JOURNAL OF MICROBIOLOGY, IMMUNOLOGY AND INFECTION vol. 37, no. 3, June 2004, pages 135 - 144, XP009166915 Retrieved from the Internet: <URL:http://pub.hato.com.tw/jmii/files/pdf/ 2004June/20040601.pdf>
- HIROMOTO YASUAKI ET AL: "Phylogenetic analysis of the three polymerase genes (PB1, PB2 and PA) of influenza B virus", JOURNAL OF GENERAL VIROLOGY, SOCIETY FOR GENERAL MICROBIOLOGY, SPENCERS WOOD, GB, vol. 81, no. 4, 1 April 2000 (2000-04-01), pages 929-937, XP002301812, ISSN: 0022-1317
- LIN Y ET AL: "Recent changes among human influenza viruses", VIRUS RESEARCH, AMSTERDAM, NL, vol. 103, no. 1-2, 1 July 2004 (2004-07-01), pages 47-52, XP026850650, ISSN: 0168-1702 [retrieved on 2004-05-25]
- Isolde C. Dapat ET AL: "Genetic Characterization of Human Influenza Viruses in the Pandemic (2009-2010) and Post-Pandemic (2010-2011) Periods in Japan", PLoS ONE, vol. 7, no. 6, 27 June 2012 (2012-06-27), page e36455, XP055084747, DOI: 10.1371/journal.pone.0036455
- 'Weekly Epidemiological Record / Relevé Epidémiologique Hebdomadaire', [Online] 01 March 1991, pages 57 - 64, XP055084824 Retrieved from the Internet: <URL:http://whqlibdoc.who.int/wer/WHO_WER_1 991/WER1991_66_57-64 (N°9).pdf> [retrieved on 2013-10-22]
- I.G. Barr ET AL: "Reassortants in recent human influenza A and B isolates from South East Asia and Oceania", Virus Research, vol. 98, no. 1, 1 December 2003 (2003-12-01), pages 35-44, XP055084829, ISSN: 0168-1702, DOI: 10.1016/j.virusres.2003.08.011
- Ian G Barr ET AL: "Circulation and antigenic drift in human influenza B viruses in SE Asia and Oceania since 2000", CDI, 1 January 2006 (2006-01-01), pages 350-357, XP055084833, Retrieved from the Internet: URL:https://www.health.gov.au/internet/mai n/publishing.nsf/Content/cda-cdi3003-pdf-c nt.htm/$FILE/cdi3003h.pdf [retrieved on 2013-10-22]
- [Online] 14 February 2006, XP055084844 Retrieved from the Internet: <URL:http://www.who.int/influenza/vaccines/ 2007northreport.pdf> [retrieved on 2013-10-22]
- X. Xu ET AL: "Multiple lineages co-circulation and genetic reassortment of the neuraminidase and hemagglutinin genes within influenza viruses of the same type/subtype", International congress series, vol. 1219, 1 October 2001 (2001-10-01), pages 383-387, XP055084837, ISSN: 0531-5131, DOI: 10.1016/S0531-5131(01)00348-X
- N. KISHIDA ET AL: "Evaluation of Influenza Virus A/H3N2 and B Vaccines on the Basis of Cross-Reactivity of Postvaccination Human Serum Antibodies against Influenza Viruses A/H3N2 and B Isolated in MDCK Cells and Embryonated Hen Eggs", CLINICAL AND VACCINE IMMUNOLOGY, vol. 19, no. 6, 4 April 2012 (2012-04-04), pages 897-908, ISSN: 1556-6811, DOI: 10.1128/CVI.05726-11
- JIRÍ BERAN ET AL: "Immunogenicity and safety of quadrivalent versus trivalent inactivated influenza vaccine: a randomized, controlled trial in adults", BMC INFECTIOUS DISEASES, BIOMED CENTRAL, LONDON, GB, vol. 13, no. 1, 20 May 2013 (2013-05-20), page 224, ISSN: 1471-2334, DOI: 10.1186/1471-2334-13-224

## Description

### TECHNICAL FIELD

This invention is in the field of vaccines for protecting against influenza virus infection, and in particular vaccines that include antigens derived from more than three viral strains.

### DISCLOSURE OF THE INVENTION

The applicant has realized that many deficiencies in current trivalent vaccines are caused by the use of eggs during their manufacture. Egg-based manufacturing is not well-suited for increasing the amount of antigen required in a particular season, in terms of both lead time and capacity. Current egg-based manufacturing requires viruses to be adapted to growth in eggs, which both adds a time-consuming phase at the beginning of manufacture and introduces a selection pressure which reduces the match between vaccine strains and circulating strains. Moreover, influenza B virus strains do not grow well in eggs, and high-growth reassortants are not available. The absolute requirement for egg growth in current procedures is well demonstrated by the H3N2 mismatch in the 2003/04 season, which mainly arose because the relevant Fuji-like strains had originally not been isolated using eggs. To overcome these deficiencies, cell culture can be used for viral growth instead of eggs. Therefore, vaccines of the invention are produced from viruses grown in cell culture rather than in eggs, as defined in the claims.

The inability of egg-based technology to increase the amount of antigen produced in a particular season could also be addressed by reducing the amount of antigen in a vaccine. One way of reducing the amount is to use an adjuvant. Therefore, vaccines of the invention include an adjuvant comprising an oil-in-water emulsion.

In some embodiments, the vaccines contain substantially the same mass of hemagglutinin (HA) for each of the influenza virus strains (*e.g.* about 1:1:1:1). The mass of HA per strain may about 15µg per dose, or in some embodiments may be less than 15µg per dose (*e.g*. less than 10µg per dose) or more than 15µg per dose (*e.g.* >20µg/dose, >25µg/dose, *etc*., such as about 30µg/dose). In other embodiments the vaccines contain different masses of HA for different strains.

In some embodiments, the vaccines are not split or whole virion inactivated vaccines, but are live or purified glycoprotein vaccines.

The invention also provides a method for preparing a vaccine of the invention comprising the steps of: (i) growing four different strains of influenza virus in cell culture; (ii) preparing an antigen composition from cach of the viruses grown in step (i): and (iii) combining the antigen compositions (*e.g.* with a pharmaceutical carrier), to give the vaccine.

### Strain selection

Influenza virus strains for use in vaccines change from season to season. In the current inter-pandemic period, trivalent vaccines include two influenza A strains (H1N1 and H3N2) and one influenza B strain. Vaccines of the invention include at least four influenza virus strains. The different strains will typically be grown separately and then mixed after the viruses have been harvested and antigens have been prepared. Thus a process of the invention may include the step of mixing antigens from more than one influenza strain.

Influenza A virus currently displays sixteen HA subtypes: H1, H2, H3, H4, H5, H6, H7, H8, H9, H10, H11, H12, H13, H14, H15 and H16. The invention may protect against one or more of influenza A virus NA subtypes N1, N2, N3, N4, N5, N6, N7, N8 or N9. In vaccines including only two influenza A virus strains, these will be one H1N1 strain and one H3N2 strain.

Influenza B virus currently does not display different HA subtypes, but influenza B virus strains do fall into two distinct lineages. These lineages emerged in the late 1980s and have HAs which can be antigenically and/or genetically distinguished from each other [3]. Current influenza B virus strains are either B/Victoria/2/87-like or B/Yamagata/16/88-like. Where a vaccine of the invention includes two influenza B strains, one B/Victoria/2/87-like strain and one B/Yamagata/16/88-like strain will be included. These strains are usually distinguished antigenically, but differences in amino acid sequences have also been described for distinguishing the two lineages *e.g.* B/Yamagata/16/88-like strains often (but not always) have HA proteins with deletions at amino acid residue 164, numbered relative to the 'Lee40' HA sequence [4].

Thus vaccines of the invention include hemagglutinins from: (i) a H1N1 strain; (ii) a H3N2 strain; (iii) a B/Victoria/2/87-like strain; and (iv) B/Yamagata/16/88-like strain.

In some embodiments of the invention, at least two of the influenza B virus strains may have distinct hemagglutinins but related neuraminidases. For instance, they may both have a B/Victoria/2/87-like neuraminidase [5] or may both have a B/Yamagata/16/88-like neuraminidase. For instance, two B/Victoria/2/87-like neuraminidases may both have one or more of the following sequence characteristics: (1) not a serine at residue 27, but preferably a leucine; (2) not a glutamate at residue 44, but preferably a lysine; (3) not a threonine at residue 46, but preferably an isoleucine; (4) not a proline at residue 51, but preferably a serine; (5) not an arginine at residue 65, but preferably a histidine; (6) not a glycine at residue 70, but preferably a glutamate; (7) not a leucine at residue 73, but preferably a phenylalanine; and/or (8) not a proline at residue 88, but preferably a glutamine. Similarly, in some embodiments the neuraminidase may have a deletion at residue 43, or it may have a threonine; a deletion at residue 43, arising from a trinucleotide deletion in the NA gene, has been reported as a characteristic of B/Victoria/2/87-like strains, although recent strains have regained Thr-43 [5]. Conversely, of course, the opposite characteristics may be shared by two B/Yamagata/16/88-like neuraminidases *e.g*. S27, E44, T46, P51, R65, G70, L73, and/or P88. These amino acids are numbered relative to the 'Lee40' neuraminidase sequence [6]. Thus a A-A-B-B vaccine of the invention may use two B strains that are antigenically distinct for HA (one B/Yamagata/16/88-like, one B/Victoria/2/87-like), but are related for NA (both B/Yamagata/16/88-like, or both B/Victoria/2/87-like).

The invention is not restricted to 4-valent vaccines, and encompasses 5-valent, 6-valent, 7-valent, *etc.* vaccines. An example 5-valent vaccine may include three influenza A strains (*e.g*. one HI strain and two H3 strains, as discussed above) plus two influenza B strains. For example, an A-A-A-B-B vaccine may include antigens (preferably hemagglutinins) from: (i) a H1N1 strain; (ii) a A/Moscow/10/99-like H3N2 strain; (iii) a A/Fujian/411/2002-like H3N2 strain; (iv) a B/Victoria/2/87-like strain; and (v) a B/Yamagata/16/88-like strain. Another A-A-A-B-B vaccine may include antigens (preferably hemagglutinins) from: (i) a H1N1 strain; (ii) a H3N2 strain; (iii) a H5 influenza A virus strain, such as a H5N1 strain; (iv) a B/Victoria/2/87-like strain; and (v) a B/Yamagata/16/88-like strain. An A-A-A-A-B-B vaccine may include antigens (preferably hemagglutinins) from: (i) a H1N1 strain; (ii) a A/Moscow/10/99-like H3N2 strain; (iii) a A/Fujian/411/2002-like H3N2 strain; (iv) a H5 influenza A virus strain, such as a H5N1 strain; (v) a B/Victoria/2/87-like strain; and (vi) a B/Yamagata/16/88-like strain.

An influenza virus used with the invention may be a reassortant strain, and may have been obtained by reverse genetics techniques. Reverse genetics techniques [e.g. 7-11] allow influenza viruses with desired genome segments to be prepared *in vitro* using plasmids. Typically, it involves expressing (a) DNA molecules that encode desired viral RNA molecules *e.g*. from poll promoters or bacteriophage RNA polymerase promoters, and (b) DNA molecules that encode viral proteins *e.g*. from polII promoters, such that expression of both types of DNA in a cell leads to assembly of a complete intact infectious virion. The DNA preferably provides all of the viral RNA and proteins, but it is also possible to use a helper virus to provide some of the RNA and proteins. Plasmid-based methods using separate plasmids for producing each viral RNA can be used [12-14], and these methods will also involve the use of plasmids to express all or some (*e.g.* just the PB1, PB2, PA and NP proteins) of the viral proteins, with up to 12 plasmids being used in some methods. To reduce the number of plasmids needed, a recent approach [15] combines a plurality of RNA polymerase 1 transcription cassettes (for viral RNA synthesis) on the same plasmid (*e.g*. sequences encoding 1, 2, 3, 4, 5, 6, 7 or all 8 influenza A vRNA segments), and a plurality of protein-coding regions with RNA polymerase II promoters on another plasmid (*e.g*, sequences encoding 1, 2, 3, 4, 5, 6, 7 or all 8 influenza A mRNA transcripts). Preferred aspects of the reference 15 method involve: (a) PB1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

As an alternative to using poll promoters to encode the viral RNA segments, it is possible to use bacteriophage polymerase promoters [16]. For instance, promoters for the SP6, T3 or T7 polymerases can conveniently be used. Because of the species-specificity of poll promoters, bacteriophage polymerase promoters can be more convenient for many cell types (*e.g*. MDCK), although a cell must also be transfected with a plasmid encoding the exogenous polymerase enzyme.

In other techniques it is possible to use dual poll and polII promoters to simultaneously code for the viral RNAs and for expressible mRNAs from a single template [17,18].

Thus an influenza A virus may include one or more RNA segments from a A/PR/8/34 virus (typically 6 segments from A/PR/8/34, with the HA and N segments being from a vaccine strain, *i.e.* a 6:2 reassortant). It may also include one or more RNA segments from a A/WSN/33 virus, or from any other virus strain useful for generating reassortant viruses for vaccine preparation. An influenza A virus may include fewer than 6 (*i.e.* 0, 1, 2, 3, 4 or 5) viral segments from an AA/6/60 influenza virus (A/Ann Arbor/6/60). An influenza B virus may include fewer than 6 (*i.e*. 0, 1, 2, 3, 4 or 5) viral segments from an AA/1/66 influenza virus (B/Ann Arbor/1/66). Typically, the invention protects against a strain that is capable of human-to-human transmission, and so the strain's genome will usually include at least one RNA segment that originated in a mammalian (*e.g*. in a human) influenza virus. It may include NS segment that originated in an avian influenza virus.

Strains whose antigens can be included in the compositions may be resistant to antiviral therapy (*e.g.* resistant to oseltamivir [19] and/or zanamivir), including resistant pandemic strains [20].

The strains have not been passaged through eggs at any stage between isolation from a patient and replication in a cell culture system, inclusive. The use exclusively of MDCK cells for all steps from isolation to virus replication is one preferred embodiment of the invention.

In some embodiments, strains used with the invention will thus have hemagglutinin with a binding preference for oligosaccharides with a Sia(α2,6)Gal terminal disaccharide compared to oligosaccharides with a Sia(α2,3)Gal terminal disaccharide. Human influenza viruses bind to receptor oligosaccharides having a Sia(α2,6)Gal terminal disaccharide (sialic acid linked α-2,6 to galactose), but eggs and Vero cells have receptor oligosaccharides with a Sia(α2,3)Gal terminal disaccharide. Growth of human influenza viruses in cells such as MDCK provides selection pressure on hemagglutinin to maintain the native Sia(α2,6)Gal binding, unlike egg passaging.

To determine if a virus has a binding preference for oligosaccharides with a Sia(α2,6)Gal terminal disaccharide compared to oligosaccharides with a Sia(α2,3)Gal terminal disaccharide, various assays can be used. For instance, reference 21 describes a solid-phase enzyme-linked assay for influenza virus receptor-binding activity which gives sensitive and quantitative measurements of affinity constants. Reference 22 used a solid-phase assay in which binding of viruses to two different sialylglycoproteins was assessed (ovomucoid, with Sia(α2,3)Gal determinants; and pig α₂-macroglobulin, which Sia(α2,6)Gal determinants), and also describes an assay in which the binding of virus was assessed against two receptor analogs: free sialic acid (Neu5Ac) and 3'-sialyllactose (Neu5Acα2-3Galβ-4Glc). Reference 23 reports an assay using a glycan array which was able to clearly differentiate receptor preferences for α2,3 or α2,6 linkages. Reference 24 reports an assay based on agglutination of human erythrocytes enzymatically modified to contain either Sia(α2,6)Gal or Sia(α2,3)Gal. Depending on the type of assay, it may be performed directly with the virus itself, or can be performed indirectly with hemagglutinin purified from the virus.

In some embodiments influenza strains used with the invention have glycoproteins (including hemagglutinin) with a different glycosylation pattern from egg-derived viruses. Thus the glycoproteins will include glycoforms that are not seen in chicken eggs.

In some embodiments, the invention does not use a combination of A/Singapore/6/86(H1N1), A/Beijing/353/89(H3N2), B/Beijing/1/87, and B/Panama/45/90 strains (and in particular not a combination of split antigens from these four strains, and/or not with 15µg HA per strain). In other embodiments, the invention does not use a combination of A/Taiwan/1/86(H1N1), A/Guizhou/54/89(H3N2), B/Beijing/1/87 and B/Yamagata/16/88 strains (and in particular not a combination of whole virions from these four strains).

### Vaccine preparation

Various forms of influenza virus vaccine are currently available, and vaccines are generally based either on live virus or on inactivated virus. Inactivated vaccines may be based on whole virions, 'split' virions, or on purified surface antigens. Influenza antigens can also be presented in the form of virosomes. The invention can used with any of these types of vaccine.

Existing live vaccines include MedImmune's FLUMIST™ product (trivalent live virus). Vaccine is prepared by a process that comprises growing the virus on a suitable substrate and then purifying virions from virion-containing fluids. For example, the fluids may be clarified by centrifugation, and stabilized with buffer (*e.g.* containing sucrose, potassium phosphate, and monosodium glutamate).

Where an inactivated virus is used, the vaccine may comprise whole virion, split virion, or purified surface antigens (including hemagglutinin and, usually, also including neuraminidase). Chemical means for inactivating a virus include treatment with an effective amount of one or more of the following agents: detergents, formaldehyde, β-propiolactone, methylene blue, psoralen, carboxyfullerene (C60), binary ethylamine, acetyl ethyleneimine, or combinations thereof. Non-chemical methods of viral inactivation are known in the art, such as for example UV light or gamma irradiation.

Virions can be harvested from virus-containing fluids by various methods. For example, a purification process may involve zonal centrifugation using a linear sucrose gradient solution that includes detergent to disrupt the virions. Antigens may then be purified, after optional dilution, by diafiltration.

Split virions are obtained by treating purified virions with detergents (*e.g*. ethyl ether, polysorbate 80, deoxycholate, tri-*N*-butyl phosphate, Triton X-100, Triton N101, cetyltrimethylammonium bromide, Tergitol NP9, *etc*.) to produce subvirion preparations, including the 'Tween-ether' splitting process. Methods of splitting influenza viruses are well known in the art *e.g.* see refs. 25-30, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (*e.g*. cationic) surfactants *e.g*. alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, quaternary ammonium compounds, sarcosyl, CTABs (cetyl trimethyl ammonium bromides), tri-*N*-butyl phosphate, Cetavlon, myristyltrimethylammonium salts, lipofectin, lipofectamine, and DOT-MA, the octyl- or nonylphenoxy polyoxyethanols (*e.g.* the Triton surfactants, such as Triton X-100 or Triton N101), polyoxyethylene sorbitan esters (the Tween surfactants), polyoxyethylene ethers, polyoxyethlene esters, *etc*. One useful splitting procedure uses the consecutive effects of sodium deoxycholate and formaldehyde, and splitting can take place during initial virion purification (*e.g*. in a sucrose density gradient solution). Thus a splitting process can involve clarification of the virion-containing material (to remove non-virion material), concentration of the harvested virions (*e.g*. using an adsorption method, such as CaHPO₄ adsorption), separation of whole virions from non-virion material, splitting of virions using a splitting agent in a density gradient centrifugation step (*e.g*. using a sucrose gradient that contains a splitting agent such as sodium deoxycholate), and then filtration (*e.g*. ultrafiltration) to remove undesired materials. Split virions can usefully be resuspended in sodium phosphate-buffered isotonic sodium chloride solution. The BEGRIVAC™, FLUARIX™, FLUZONE™ and FLUSHIELD™ products are split vaccines.

Purified surface antigen vaccines comprise the influenza surface antigens haemagglutinin and, typically, also neuraminidase. Processes for preparing these proteins in purified form are well known in the art. The FLUVIRIN™, AGRIPPAL™ and INFLUVAC™ products are subunit vaccines.

Another form of inactivated influenza antigen is the virosome [31] (nucleic acid free viral-like liposomal particles). Virosomes can be prepared by solubilization of influenza virus with a detergent followed by removal of the nucleocapsid and reconstitution of the membrane containing the viral glycoproteins. An alternative method for preparing virosomes involves adding viral membraneglycoproteins to excess amounts of phospholipids, to give liposomes with viral proteins in their membrane. The invention can be used to store bulk virosomes as in the INFLEXAL V™ and INVAVAC™ products.

The influenza virus may be attenuated. The influenza virus may be temperature-sensitive. The influenza virus may be cold-adapted. These three features arc particularly useful when using live virus as an antigen.

HA is the main immunogen in current inactivated influenza vaccines, and vaccine doses arc standardised by reference to HA levels, typically measured by SRID. Existing vaccines typically contain about 15µg of HA per strain, although lower doses can be used *e.g*. for children, or in pandemic situations, or when using an adjuvant. Fractional doses such as ½ (*i.e.* 7.5µg HA per strain), ¼ and ⅛ have been used [68,69], as have higher doses (*e.g*. 3x or 9x doses [32,33]). Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc*. Particular doses include *e.g*. about 45, about 30, about 15, about 10, about 7.5, about 5, about 3.8, about 1.9, about 1.5, *etc.* per strain. It is preferred to use substantially the same mass of HA for each strain included in the vaccine *e.g*. such that the HA mass for each strain is within 10% of the mean HA mass per strain, and preferably within 5% of the mean.

For live vaccines, dosing is measured by median tissue culture infectious dose (TCID₅₀) rather than HA content, and a TCID₅₀ of between 10⁶ and 10⁸ (preferably between 10⁶⁻⁵-10^{7.5}) per strain is typical.

Strains used with the invention may have a natural HA as found in a wild-type virus, or a modified HA. For instance, it is known to modify HA to remove determinants (*e.g*. hyper-basic regions around the HA1/HA2 cleavage site) that cause a virus to be highly pathogenic in avian species. The HAs of influenza B viruses used with the invention preferably have Asn at amino acid 197, providing a glycosylation site [34].

### Cell lines

Rather than use SPF eggs as the substrate for viral growth, the invention uses cell lines that support influenza virus replication. The cell line will typically be of mammalian origin. Suitable mammalian cells of origin include, but are not limited to, hamster, cattle, primate (including humans and monkeys) and dog cells, although the use of primate cells is not preferred. Various cell types may be used, such as kidney cells, fibroblasts, retinal cells, lung cells, *etc*. Examples of suitable hamster cells are the cell lines having the names BHK21 or HKCC. Suitable monkey cells are *e.g*. African green monkey cells, such as kidney cells as in the Vero cell line [35-37]. Suitable dog cells are *e.g*. kidney cells, as in the CLDK and MDCK cell lines.

Thus suitable cell lines include, but are not limited to: MUCK; CHO; CLDK; HKCC; 293T; BHK; Vero; MRC-5; PER.C6 [38]; FRhL2; WI-38; *etc*. Suitable cell lines are widely available *e.g*. from the American Type Cell Culture (ATCC) collection [39], from the Coriell Cell Repositories [40], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940.

The most preferred cell lines are those with mammalian-type glycosylation. As a less-preferred alternative to mammalian cell lines, virus can be grown on avian cell lines [*e.g*. refs. 41-43], including cell lines derived from ducks (*e.g*. duck retina) or hens. Examples of avian cell lines include avian embryonic stem cells [41,44] and duck retina cells [42]. Suitable avian embryonic stem cells, include the EBx cell line derived from chicken embryonic stem cells, EB45, EB14, and EB 14-074 [45]. Chicken embryo fibroblasts (CEF) may also be used. Rather than using avian cells, however, the use of mammalian cells means that vaccines can be free from avian DNA and egg proteins (such as ovalbumin and ovomucoid), thereby reducing allergenicity.

The most preferred cell lines for growing influenza viruses are MDCK cell lines [46-49], derived from Madin Darby canine kidney. The original MDCK cell line is available from the ATCC as CCL-34, but derivatives of this cell line may also be used. For instance, reference 46 discloses a MDCK cell line that was adapted for growth in suspension culture ('MDCK 33016', deposited as DSM ACC 2219). Similarly, reference 50 discloses a MDCK-derived cell line that grows in suspension in serum-free culture ('B-702', deposited as FERM BP-7449). Reference 51 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 52 discloses MDCK cell lines with high susceptibility to infection, including 'MDCK.5F1' cells (ATCC CRL-12042). Any of these MDCK cell lines can be used.

Virus may be grown on cells in adherent culture or in suspension. Microcarrier cultures can also be used. In some embodiments, the cells may thus be adapted for growth in suspension.

Cell lines are preferably grown in serum-free culture media and/or protein free media. A medium is referred to as a serum-free medium in the context of the present invention in which there are no additives from serum of human or animal origin. The cells growing in such cultures naturally contain proteins themselves, but a protein-free medium is understood to mean one in which multiplication of the cells occurs with exclusion of proteins, growth factors, other protein additives and non-serum proteins, but can optionally include proteins such as trypsin or other proteases that may be necessary for viral growth.

Cell lines supporting influenza virus replication are preferably grown below 37°C [53] (*e.g*. 30-36°C, or at about 30°C, 31 °C, 32°C, 33°C, 34°C, 35°C, 36°C) during viral replication.

Methods for propagating influenza virus in cultured cells generally includes the steps of inoculating a culture of cells with an inoculum of the strain to be grown, cultivating the infected cells for a desired time period for virus propagation, such as for example as determined by virus titer or antigen expression (*e.g*. between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (*e.g*. monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture *e.g*. before inoculation, at the same time as inoculation, or after inoculation [53].

In preferred embodiments, particularly with MDCK cells, a cell line is not passaged from the master working cell bank beyond 40 population-doubling levels.

The viral inoculum and the viral culture are preferably free from (*i.e*. will have been tested for and given a negative result for contamination by) herpes simplex virus, respiratory syncytial virus, parainfluenza virus 3, SARS coronavirus, adenovirus, rhinovirus, reoviruses, polyomaviruses, bimaviruses, circoviruses, and/or parvoviruses [54]. Absence of herpes simplex viruses is particularly preferred.

### Host cell DNA

Where virus has been grown on a cell line then it is standard practice to minimize the amount of residual cell line DNA in the final vaccine, in order to minimize any oncogenic activity of the DNA.

Thus a vaccine composition prepared according to the invention preferably contains less than 10ng (preferably less than 1ng, and more preferably less than 100pg) of residual host cell DNA per dose, although trace amounts of host cell DNA may be present.

Vaccines containing <10ng (*e.g.* <1ng, <100pg) host cell DNA per 15µg of haemagglutinin are preferred, as are vaccines containing <10ng (*e.g*. <1ng, <100pg) host cell DNA per 0.25ml volume. Vaccines containing <10ng (*e.g*. <1ng, <100pg) host cell DNA per 50µg of haemagglutinin are more preferred, as are vaccines containing <10ng (*e.g.* <1ng, <100pg) host cell DNA per 0.5ml volume.

It is preferred that the average length of any residual host cell DNA is less than 500bp *e.g.* less than 400bp, less than 300bp, less than 200bp, less than 100bp, *etc.*

Contaminating DNA can be removed during vaccine preparation using standard purification procedures *e.g.* chromatography, *etc.* Removal of residual host cell DNA can be enhanced by nuclease treatment *e.g*. by using a DNase. A convenient method for reducing host cell DNA contamination is disclosed in references 55 & 56, involving a two-step treatment, first using a DNase (*e.g*. Benzonase), which may be used during viral growth, and then a cationic detergent (*e.g*. CTAB), which may be used during virion disruption. Removal by β-propiolactone treatment can also be used.

Measurement of residual host cell DNA is now a routine regulatory requirement for biologicals and is within the normal capabilities of the skilled person. The assay used to measure DNA will typically be a validated assay [57,58]. The performance characteristics of a validated assay can be described in mathematical and quantifiable terms, and its possible sources of error will have been identified. The assay will generally have been tested for characteristics such as accuracy, precision, specificity. Once an assay has been calibrated (*e.g*. against known standard quantities of host cell DNA) and tested then quantitative DNA measurements can be routinely performed. Three main techniques for DNA quantification can be used: hybridization methods, such as Southern blots or slot blots [59]; immunoassay methods, such as the Threshold™ System [60]; and quantitative PCR [61]. These methods are all familiar to the skilled person, although the precise characteristics of each method may depend on the host cell in question *e.g*. the choice of probes for hybridization, the choice of primers and/or probes for amplification, *etc.* The Threshold™ system from *Molecular Devices* is a quantitative assay for picogram levels of total DNA, and has been used for monitoring levels of contaminating DNA in biopharmaceuticals [60]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA *e.g*. AppTec™ Laboratory Services, BioReliance™, Althea Technologies, *etc.* A comparison of a chemiluminescent hybridisation assay and the total DNA Threshold™ system for measuring host cell DNA contamination of a human viral vaccine can be found in reference 62.

### Pharmaceutical compositions

Compositions of the invention are pharmaceutically acceptable. They usually include components in addition to the antigens *e.g*. they typically include one or more pharmaceutical carrier(s) and/or excipient(s). As described below, an oil-in-water emulsion adjuvant is included. A thorough discussion of such components is available in reference 63.

Compositions will generally be in aqueous form.

The composition may include preservatives such as thiomersal or 2-phenoxyethanol. It is preferred, however, that the vaccine should be substantially free from (*i.e.* less than 5µg/ml) mercurial material *e.g*. thiomersal-free [29,64]. Vaccines containing no mercury are more preferred. Preservative-free vaccines are particularly preferred. α-tocopherol succinate can be included as an alternative to mercurial compounds [29].

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [65], but keeping osmolality in this range is nevertheless preferred.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The pH of a composition will generally be between 5.0 and 8.1, and more typically between 6.0 and 8.0 *e.g*. 6.5 and 7.5, or between 7.0 and 7.8. A process of the invention may therefore include a step of adjusting the pH of the bulk vaccine prior to packaging.

The composition is preferably sterile. The composition is preferably non-pyrogenic *e.g*. containing <1 EU (endotoxin unit, a standard measure) per dose, and preferably <0.1 EU per dose. The composition is preferably gluten free.

Compositions of the invention may include detergent *e.g*. a polyoxyethylene sorbitan ester surfactant (known as 'Tweens'), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts. Thus the vaccine may included less than 1mg/ml of each of octoxynol-10 and polysorbate 80. Other residual components in trace amounts could be antibiotics (*e.g*. neomycin, kanamycin, polymyxin B).

The composition may include material for a single immunisation, or may include material for multiple immunisations (*i.e.* a 'multidose' kit). The inclusion of a preservative is preferred in multidose arrangements. As an alternative (or in addition) to including a preservative in multidose compositions, the compositions may be contained in a container having an aseptic adaptor for removal of material.

Influenza vaccines are typically administered in a dosage volume of about 0.5ml, although a half dose (*i.e.* about 0.25ml) may be administered to children.

Compositions and kits are preferably stored at between 2°C and 8°C. They should not be frozen. They should ideally be kept but of direct light.

### Adjuvants

Compositions of the invention include an adjuvant comprising an oil-in-water emulsion, which can function to enhance the immune responses (humoral and/or cellular) elicited in a patient who receives the composition. The use of adjuvants with influenza vaccines has been described before. In references 66 & 67, aluminum hydroxide was used, and in reference 68, a mixture of aluminum hydroxide and aluminum phosphate was used. Reference 69 also described the use of aluminum salt adjuvants. The FLUAD™ product from Chiron Vaccines includes an oil-in-water emulsion.

Compositions may include two or more adjuvants. For example, they may advantageously include both an oil-in-water emulsion and a cytokine-inducing agent, as this combination improves the cytokine responses elicited by influenza vaccines, such as the interferon-y response, with the improvement being much greater than seen when either the emulsion or the agent is used on its own.

Antigens and adjuvants in a composition will typically be in admixture.

### Oil-in-water emulsion adjuvants

Oil-in-water emulsions have been found to be particularly suitable for use in adjuvanting influenza virus vaccines. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g*. obtained from the jojoba bean. Seed oils include safflower oil, cottonseed oil, sunflower seed oil, sesame seed oil, *etc.* In the grain group, corn oil is the most readily available, but the oil of other cereal grains such as wheat, oats, rye, rice, teff, triticale, *etc.* may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. Preferred surfactants for including in the emulsion are Tween 80 (polyoxyethylene sorbitan monooleate), Span 85 (sorbitan trioleate), lecithin and Triton X-100.

Mixtures of surfactants can be used *e.g.* Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to I % or about 0.5%.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [94-96], as described in more detail in Chapter 10 of ref. 97 and chapter 12 of ref. 98. The MF59 emulsion advantageously includes citrate ions *e.g*. 10mM sodium citrate buffer.
- An emulsion of squalene, a tocopherol, and Tween 80. The emulsion may include phosphate buffered saline. It may also include Span 85 (*e.g*. at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. Squalene and Tween 80 may be present volume ratio of about 5:2. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g*. with an average diameter of between 100 and 250nm, preferably about 180nm.

- An emulsion of squalene, a tocopherol, and a Triton detergent (*e.g*. Triton X-100). The emulsion may also include a 3d-MPL (see below). The emulsion may contain a phosphate buffer.
- An emulsion comprising a polysorbate (*e.g*. polysorbate 80), a Triton detergent (*e.g*. Triton X-100) and a tocopherol (*e.g*. an α-tocopherol succinate). The emulsion may include these three components at a mass ratio of about 75:11:10 (*e.g*. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [99] (0.05-1% Thr-MDP, 5% squalane, 2.5% Pluronic L121 and 0.2% polysorbate 80). It can also be used without the Thr-MDP, as in the "AF" adjuvant [100] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant (*e.g*. polyoxyethylene (12) cetostearyl ether) and a hydrophobic nonionic surfactant (*e.g*. a sorbitan ester or mannide ester, such as sorbitan monoleate or 'Span 80'). The emulsion is preferably thermoreversible and/or has at least 90% of the oil droplets (by volume) with a size less than 200 nm [101]. The emulsion may also include one or more of: alditol; a cryoprotective agent (*e.g*. a sugar, such as dodecylmaltoside and/or sucrose); and/or an alkylpolyglycoside. Such emulsions may be lyophilized.
- An emulsion of squalene, poloxamer 105 and Abil-Care [102]. The final concentration (weight) of these components in adjuvanted vaccines are 5% squalene, 4% poloxamer 105 (pluronic polyol) and 2% Abil-Care 85 (Bis-PEG/PPG-16/16 PEG/PPG-16/16 dimethicone; caprylic/capric triglyceride).
- An emulsion having from 0.5-50% of an oil, 0.1-10% of a phospholipid, and 0.05-5% of a non-ionic surfactant. As described in reference 103, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 104, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin (*e.g.* QuilA or QS21) and a sterol (*e.g.* a cholesterol) are associated as helical micelles [105].
- An emulsion comprising a mineral oil, a non-ionic lipophilic ethoxylated fatty alcohol, and a non-ionic hydrophilic surfactant (*e.g*. an ethoxylated fatty alcohol and/or polyoxyethylene-polyoxypropylene block copolymer) [106].
- An emulsion comprising a mineral oil, a non-ionic hydrophilic ethoxylated fatty alcohol, and a non-ionic lipophilic surfactant (*e.g*. an ethoxylated fatty alcohol and/or polyoxyethylene-polyoxypropylene block copolymer) [106].

The emulsions may be mixed with antigen extemporaneously, at the time of delivery. Thus the adjuvant and antigen may be kept separately in a packaged or distributed vaccine, ready for final formulation at the time of use. The antigen will generally be in an aqueous form, such that the vaccine is finally prepared by mixing two liquids. The volume ratio of the two liquids for mixing can vary (*e.g*. between 5:1 and 1:5) but is generally about 1:1.

After the antigen and adjuvant have been mixed, haemagglutinin antigen will generally remain in aqueous solution but may distribute itself around the oil/water interface. In general, little if any haemagglutinin will enter the oil phase of the emulsion.

Where a composition includes a tocopherol, any of the α, β, γ, δ, ε or ξ tocopherols can be used, but α-tocopherols are preferred. The tocopherol can take several forms *e.g*. different salts and/or isomers. Salts include organic salts, such as succinate, acetate, nicotinate, *etc*. D-α-tocopherol and DL-α-tocopherol can both be used. Tocopherols are advantageously included in vaccines for use in elderly patients (*e.g*. aged 60 years or older) because vitamin E has been reported to have a positive effect on the immune response in this patient group [107]. They also have antioxidant properties that may help to stabilize the emulsions [108]. A preferred α-tocopherol is DL-α-tocopherol, and the preferred salt of this tocopherol is the succinate. The succinate salt has been found to cooperate with TNF-related ligands *in vivo.* Moreover, α-tocopherol succinate is known to be compatible with influenza vaccines and to be a useful preservative as an alternative to mercurial compounds [29].

### Cytokine-inducing agents

Cytokine-inducing agents for inclusion in compositions of the invention are able, when administered to a patient, to elicit the immune system to release cytokines, including interferons and interleukins. Cytokine responses are known to be involved in the early and decisive stages of host defense against influenza infection [109]. Preferred agents can elicit the release of one or more of: interferon-γ; interleukin-1; interleukin-2; interleukin-12; TNF-α; TNF-β; and GM-CSF. Preferred agents elicit the release of cytokines associated with a Th1-type immune response *e.g*. interferon-γ, TNF-α, interleukin-2. Stimulation of both interferon-γ and interleukin-2 is preferred.

As a result of receiving a composition of the invention, therefore, a patient will have T cells that, when stimulated with an influenza antigen, will release the desired cytokine(s) in an antigen-specific manner. For example, T cells purified form their blood will release γ-interferon when exposed *in vitro* to influenza virus haemagglutinin. Methods for measuring such responses in peripheral blood mononuclear cells (PBMC) are known in the art, and include ELISA, ELISPOT, flow-cytometry and real-time PCR. For example, reference 110 reports a study in which antigen-specific T cell-mediated immune responses against tetanus toxoid, specifically γ-interferon responses, were monitored, and found that ELISPOT was the most sensitive method to discriminate antigen-specific TT-induced responses from spontaneous responses, but that intracytoplasmic cytokine detection by flow cytometry was the most efficient method to detect re-stimulating effects.

Suitable cytokine-inducing agents include, but are not limited to:
- An immunostimulatory oligonucleotide, such as one containing a CpG motif (a dinucleotide sequence containing an unmethylated cytosine linked by a phosphate bond to a guanosine), or a double-stranded RNA, or an oligonucleotide containing a palindromic sequence, or an oligonucleotide containing a poly(dG) sequence.
- 3-O-deacylated monophosphoryl lipid A ('3dMPL', also known as 'MPLT™') [111-114].
- An imidazoquinoline compound, such as Imiquimod ("R-837") [115,116], Resiquimod ("R-848") [117], and their analogs; and salts thereof (*e.g*. the hydrochloride salts). Further details about immunostimulatory imidazoquinolines can be found in references 118 to 122.
- A thiosemicarbazone compound, such as those disclosed in reference 123. Methods of formulating, manufacturing, and screening for active compounds are also described in reference 123. The thiosemicarbazones are particularly effective in the stimulation of human peripheral blood mononuclear cells for the production of cytokines, such as TNF-α.
- A tryptanthrin compound, such as those disclosed in reference 124. Methods of formulating, manufacturing, and screening for active compounds are also described in reference 124. The thiosemicarbazones are particularly effective in the stimulation of human peripheral blood mononuclear cells for the production of cytokines, such as TNF-α.
- A nucleoside analog, such as: (a) Isatorabine (ANA-245; 7-thia-8-oxoguanosine): 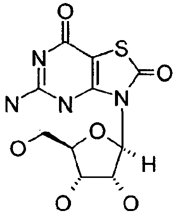 and prodrugs thereof; (b) ANA975; (c) ANA-025-1; (d) ANA380; (e) the compounds disclosed in references 125 to 127; (f) a compound having the formula: 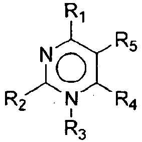 wherein:
   R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
   R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
   R₄ and R₅ are each independently H, halo, heterocyclyl, substituted heterocyclyl, -C(O)-R_{d}, C₁₋₆ alkyl, substituted C₁₋₆ alkyl, or bound together to form a 5 membered ring as in R₄₋₅: 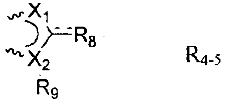 the binding being achieved at the bonds indicated by a 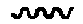
   X₁ and X₂ are each independently N, C, O, or S;
   R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚRₑ, or -C(O)-R_{d};
   R₉ is H, C₁₋₆ alkyl, substituted C₁₋₆ alkyl, heterocyclyl, substituted heterocyclyl or R₉ₐ, wherein R₉ₐ is: 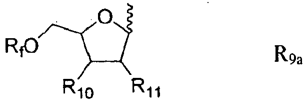 the binding being achieved at the bond indicated by a 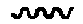
   R₁₀ and R₁₁ are each independently H, halo, C₁₋₆ alkoxy, substituted C₁₋₆ alkoxy,-NRₐR_{b}, or -OH;
   each Rₐ and R_{b} is independently H, C₁₋₆ alkyl, substituted C₁₋₆ alkyl, -C(O)R_{d}, C₆₋₁₀ aryl;
   each R_{c} is independently H, phosphate, diphosphate, triphosphate, C₁₋₆ alkyl, or substituted C₁₋₆ alkyl;
   each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), -NH(substituted C₁₋₆ alkyl), -N(C₁₋₆ alkyl)₂, -N(substituted C₁₋₆ alkyl)₂, C₆₋₁₀ aryl, or heterocyclyl;
   each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
   each R_{f} is independently H, C₁₋₆ alkyl, substituted C₁₋₆ alkyl, -C(O)R_{d}, phosphate, diphosphate, or triphosphate;
   each n is independently 0, 1, 2, or 3;
   each p is independently 0, 1, or 2; or
   or (g) a pharmaceutically acceptable salt of any of (a) to (f), a tautomer of any of (a) to (f), or a pharmaceutically acceptable salt of the tautomer.
- Loxoribine (7-allyl-8-oxoguanosine) [128].
- Compounds disclosed in reference 129, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [130,131], Hydrapthalamide compounds, Benzophenone compounds, Isoxazole compounds, Sterol compounds, Quinazilinone compounds, Pyrrole compounds [132], Anthraquinone compounds, Quinoxaline compounds, Triazine compounds, Pyrazalopyrimidine compounds, and Benzazole compounds [133].
- A polyoxidonium polymer [134,135] or other N-oxidized polyethylene-piperazine derivative.
- Compounds disclosed in reference 136.
- An aminoalkyl glucosaminide phosphate derivative, such as RC-529 [137,138].
- A phosphazene, such as poly[di(carboxylatophenoxy)phosphazene] ("PCPP") as described, for example, in references 139 and 140.
- A CD1d ligand, such as an α-glycosylceramide [141-148] (*e.g*. α-galactosylceramide), phytosphingosine-containing α-glycosylceramides, OCH, KRN7000 [(2S,3S,4R)-1-O-(α-D-galactopyranosyl)-2-(N-hexacosanoylamino)-1,3,4-octadecanetriol], CRONY-101, 3"-O-sulfo-galactosylceramide, *etc.*
- Small molecule immunopotentiators (SMIPs) such as:
   N2-methyl-1-(2-methylpropyl)-1H-imidazo[4,5-c]quinoline-2,4-diamine
   N2,N2-dimethyl-1-(2-methylpropyl)-1H-imidazo[4,5-c]quinoline-2,4-diamine
   N2-ethyl-N2-methyl-1-(2-methylpropyl)-1H-imidazo[4,5-c]quinoline-2,4-diamine
   N2-methyl-1-(2-methylpropyl)-N2-propyl-1H-imidazo[4,5-c]quinoline-2,4-diamine
   1-((2-methylpropyl)-N2-propyl-1H-imidazo[4,5-c]quinoline-2,4-diamine
   N2-butyl-1-(2-methylpropyl)-1H-imidazo[4,5-c]quinoline-2,4-diamine
   N2-butyl-N2-methyl-1-(2-methylpropyl)-1H-imidazo[4,5-c]quinoline-2,4-diamine
   N2-methyl-1-(2-methylpropyl)-N2-pentyl-1H-imidazo[4,5-c]quinoline-2,4-diamine
   N2-methyl-1-(2-methylpropyl)-N2-prop-2-enyl-1H-imidazo[4,5-c]quinoline-2,4-diamine
   1-((2-methylpropyl)-2-[(phenylmethyl)thio]-1H-imidazo[4,5-c]quinolin-4-amine
   1-((2-methylpropyl)-2-(propylthio)-1H-imidazo[4,5-c]quinolin-4-amine
   2-[[4-amino-1-(2-methylpropyl)-1H-imidazo[4,5-c]quinolin-2-yl](methyl)amino]ethanol
   2-[[4-amino-]-(2-methylpropyl)-1H-imidazo[4,5-c]quinolin-2-yl](methyl)amino]ethyl acetate
   4-amino-1-(2-methylpropyl)-1,3-dihydro-2H-imidazo[4,5-c]quinolin-2-one
   N2-butyl-1-(2-methylpropyl)-N4,N4-bis(phenylmethyl)-1H-imidazo[4,5-c]quinoline-2,4-diamine
   N2-butyl-N2-methyl-1-(2-methylpropyl)-N4,N4-bis(phenylmethyl)-1H-imidazo[4,5-c]quinoline-2,4-diamine
   N2-methyl-1-(2-methylpropyl)-N4,N4-bis(phenylmethyl)-1H-imidazo[4,5-c]quinoline-2,4-diamine
   N2,N2-dimethyl-1-(2-methylpropyl)-N4,N4-bis(phenylmethyl)-1H-imidazo[4,5-c]quinoline-2,4-diamine
   1-{4-amino-2-[methyl(propyl)amino]-1H-imidazo[4,5-c]quinolin-1-yl}-2-methylpropan-2-ol
   1-[4-amino-2-(propylamino)-1H-imidazo[4,5-c]quinolin-1-yl]-2-methylpropan-2-ol
   N4,N4-dibenzyl-1-(2-methoxy-2-methylpropyl)-N2-propyl-1H-imidazo[4,5-c]quinoline-2,4-diamine.

The cytokine-inducing agents for use in the present invention may be modulators and/or agonists of Toll-Like Receptors (TLR). For example, they may be agonists of one or more of the human TLR I, TLR2, TLR3, TLR4, TLR7, TLR8, and/or TLR9 proteins. Preferred agents are agonists of TLR7 (*e.g.* imidazoquinolines) and/or TLR9 (*e.g*. CpG oligonucleotides). These agents are useful for activating innate immunity pathways.

The cytokine-inducing agent can be added to the composition at various stages during its production. For example, it may be within an antigen composition, and this mixture can then be added to an oil-in-water emulsion. As an alternative, it may be within an oil-in-water emulsion, in which case the agent can either be added to the emulsion components before emulsification, or it can be added to the emulsion after emulsifcation. Similarly, the agent may be coacervated within the emulsion droplets. The location and distribution of the cytokine-inducing agent within the final composition will depend on its hydrophilic/lipophilic properties *e.g*. the agent can be located in the aqueous phase, in the oil phase, and/or at the oil-water interface.

The cytokine-inducing agent can be conjugated to a separate agent, such as an antigen (*e.g.* CRM197). A general review of conjugation techniques for small molecules is provided in ref. 149. As an alternative, the adjuvants may be non-covalently associated with additional agents, such as by way of hydrophobic or ionic interactions.

Two preferred cytokine-inducing agents are (a) immunostimulatory oligonucleotides and (b) 3dMPL.

Immunostimulatory oligonucleotides can include nucleotide modifications/analogs such as phosphorothioate modifications and can be double-stranded or (except for RNA) single-stranded. References 150, 151 and 152 disclose possible analog substitutions *e.g*. replacement of guanosine with 2'-deoxy-7-deazaguanosine. The adjuvant effect of CpG oligonucleotides is further discussed in refs. 153-158. A CpG sequence may be directed to TLR9, such as the motif GTCGTT or TTCGTT [159]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 160-162. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 159 & 163-165. A useful CpG adjuvant is CpG7909, also known as ProMune™·(Coley Pharmaceutical Group, Inc.).

As an alternative, or in addition, to using CpG sequences, TpG sequences can be used [166]. These oligonucleotides may be free from unmethylated CpG motifs.

The immunostimulatory oligonucleotide may be pyrimidine-rich. For example, it may comprise more than one consecutive thymidine nucleotide (e.g. TTTT, as disclosed in ref. 166), and/or it may have a nucleotide composition with >25% thymidine (*e.g.* >35%, >40%, >50%, >60%, >80%, *etc*.). For example, it may comprise more than one consecutive cytosine nucleotide (*e.g*. CCCC, as disclosed in ref. 166), and/or it may have a nucleotide composition with >25% cytosine (*e.g*. >35%, >40%, >50%, >60%, >80%, *etc*.). These oligonucleotides may be free from unmethylated CpG motifs. Immunostimulatory oligonucleotides will typically comprise at least 20 nucleotides. They may comprise fewer than 100 nucleotides.

A particularly useful adjuvant based around immunostimulatory oligonucleotides is known as IC31™ [167]. Thus an adjuvant used with the invention may comprise a mixture of (i) an oligonucleotide (*e.g*. between 15-40 nucleotides) including at least one (and preferably multiple) Cpl motifs, and (ii) a polycationic polymer, such as an oligopeptide (*e.g*. between 5-20 amino acids) including at least one (and preferably multiple) Lys-Arg-Lys tripeptide sequence(s). The oligonucleotide may be a deoxynucleotide comprising 26-mer sequence 5'-(IC)₁₃-3' (SEQ ID NO: 1). The polycationic polymer may be a peptide comprising 11-mer amino acid sequence KLKLLLLLKLK (SEQ ID NO: 2).

3dMPL (also known as 3 de-O-acylated monophosphoryl lipid A or 3-O-desacyl-4'-monophosphoryl lipid A) is an adjuvant in which position 3 of the reducing end glucosamine in monophosphoryl lipid A has been de-acylated. 3dMPL has been prepared from a heptoseless mutant of *Salmonella minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF (see also ref. 168). Preparation of 3dMPL was originally described in reference 169.

3dMPL can take the form of a mixture of related molecules, varying by their acylation (*e.g*. having 3, 4, 5 or 6 acyl chains, which may be of different lengths). The two glucosamine (also known as 2-deoxy-2-amino-glucose) monosaccharides are N-acylated at their 2-position carbons *(i.e.* at positions 2 and 2'), and there is also O-acylation at the 3' position. The group attached to carbon 2 has formula -NH-CO-CH₂-CR¹R^{1'}. The group attached to carbon 2' has formula -NH-CO-CH₂-CR²R^{2'}. The group attached to carbon 3' has formula -O-CO-CH₂-CR³R^{3'}. A representative structure is:

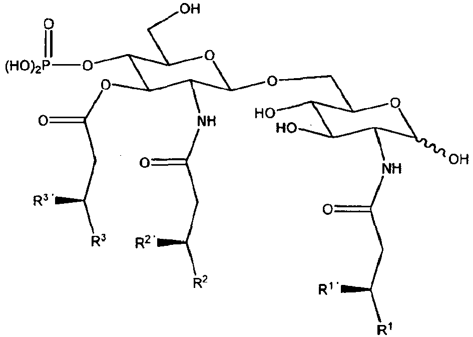

Groups R¹, R² and R³ are each independently -(CH₂)ₙ-CH₃. The value of *n* is preferably between 8 and 16, more preferably between 9 and 12, and is most preferably 10.

Groups R^{1'}, R^{2'} and R^{3'} can each independently be: (a) -H; (b) -OH; or (c) -O-CO-R⁴, where R⁴ is either -H or -(CH₂)ₘ-CH₃, wherein the value of *m* is preferably between 8 and 16, and is more preferably 10, 12 or 14. At the 2 position, *m* is preferably 14. At the 2' position, *m* is preferably 10. At the 3' position, *m* is preferably 12. Groups R^{1'}, R^{2'}, and R^{3'} are thus preferably -O-acyl groups from dodecanoic acid, tetradecanoic acid or hexadecanoic acid.

When all of R^{1'}, R^{2'} and R^{3'} are -H then the 3dMPL has only 3 acyl chains (one on each of positions 2, 2' and 3'). When only two of R^{1'}, R^{2'} and R^{3'} are -H then the 3dMPL can have 4 acyl chains. When only one of R^{1'}, R^{2'} and R^{3'} is -H then the 3dMPL can have 5 acyl chains. When none of R^{1'}, R^{2'} and R^{3'} is -H then the 3dMPL can have 6 acyl chains. The 3dMPL adjuvant used according to the invention can be a mixture of these forms, with from 3 to 6 acyl chains, but it is preferred to include 3dMPL with 6 acyl chains in the mixture, and in particular to ensure that the hexaacyl chain form makes up at least 10% by weight of the total 3dMPL *e.g*. ≥20%, ≥30%, ≥40%, ≥50% or more. 3dMPL with 6 acyl chains has been found to be the most adjuvant-active form.

Thus the most preferred form of 3dMPL for inclusion in compositions of the invention has formula (IV):

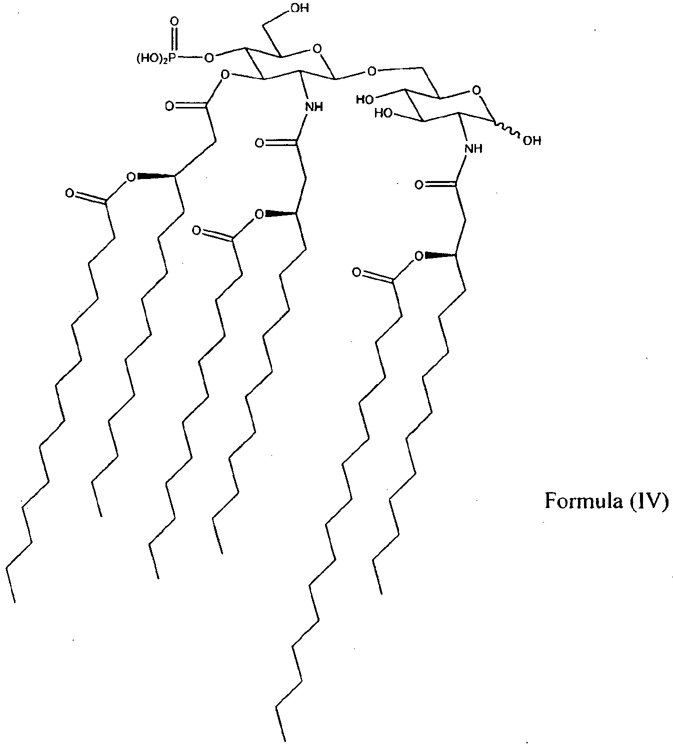

Where 3dMPL is used in the form of a mixture then references to amounts or concentrations of 3dMPL in compositions of the invention refer to the combined 3dMPL species in the mixture.

In aqueous conditions, 3dMPL can form micellar aggregates or particles with different sizes e.g. with a diameter <150nm or >500nm. Either or both of these can be used with the invention, and the better particles can be selected by routine assay. Smaller particles (e.g. small enough to give a clear aqueous suspension of 3dMPL) are preferred for use according to the invention because of their superior activity [170]. Preferred particles have a mean diameter less than 220nm, more preferably less than 200nm or less than 150nm or less than 120nm, and can even have a mean diameter less than 100nm. In most cases, however, the mean diameter will not be lower than 50nm. These particles are small enough to be suitable for filter sterilization. Particle diameter can be assessed by the routine technique of dynamic light scattering, which reveals a mean particle diameter. Where a particle is said to have a diameter ofx nm, there will generally be a distribution of particles about this mean, but at least 50% by number (e.g. ≥60%, ≥70%, ≥80%, ≥90%, or more) of the particles will have a diameter within the range *x*±25%.

3dMPL can advantageously be used in combination with an oil-in-water emulsion. Substantially all of the 3dMPL may be located in the aqueous phase of the emulsion.

### Kits of the invention

Compositions of the invention may be prepared extemporaneously, at the time of delivery, particularly when an adjuvant is being used. Thus the invention provides kits including the various components ready for mixing. The kit allows the adjuvant and the antigen to be kept separately until the time of use. This arrangement can be useful when using an oil-in-water emulsion adjuvant.

The components are physically separate from each other within the kit, and this separation can be achieved in various ways. For instance, the two components may be in two separate containers, such as vials. The contents of the two vials can then be mixed e.g. by removing the contents of one vial and adding them to the other vial, or by separately removing the contents of both vials and mixing them in a third container.

In a preferred arrangement, one of the kit components is in a syringe and the other is in a container such as a vial. The syringe can be used (*e.g*. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration.

In another preferred arrangement, the two kit components are held together but separately in the same syringe *e.g*. a dual-chamber syringe, such as those disclosed in references 182-189 *etc.* When the syringe is actuated (*e.g*. during administration to a patient) then the contents of the two chambers are mixed. This arrangement avoids the need for a separate mixing step at the time of use.

The kit components will generally be in aqueous form. In some arrangements, a component (typically an antigen component rather than an adjuvant component) is in dry form (*e.g*. in a lyophilised form), with the other component being in aqueous form. The two components can be mixed in order to reactivate the dry component and give an aqueous composition for administration to a patient. A lyophilised component will typically be located within a vial rather than a syringe. Dried components may include stabilizers such as lactose, sucrose or mannitol, as well as mixtures thereof *e.g*. lactose/sucrose mixtures, sucrose/mannitol mixtures, *etc.* One possible arrangement uses an aqueous adjuvant component in a pre-filled syringe and a lyophilised antigen component in a vial.

### Packaging of compositions or kit components

Suitable containers for compositions of the invention (or kit components) include vials, syringes (*e.g*. disposable syringes), nasal sprays, *etc..* These containers should be sterile.

Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a 'multidose' vial) *e.g*. 10 doses. Preferred vials are made of colorless glass.

A vial can have a cap (*e.g*. a Luer lock) adapted such that a pre-filled syringe can be inserted into the cap, the contents of the syringe can be expelled into the vial (*e.g*. to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Useful syringes are those marketed under the trade name "Tip-Lok"™.

Containers may be marked to show a half-dose volume *e.g*. to facilitate delivery to children. For instance, a syringe containing a 0.5ml dose may have a mark showing a 0.25ml volume.

Where a glass container (*e.g*. a syringe or a vial) is used, then it is preferred to use a container made from a borosilicate glass rather than from a soda lime glass.

A kit or composition may be packaged (*e.g*. in the same box) with a leaflet including details of the vaccine *e.g.* instructions for administration, details of the antigens within the vaccine, *etc.* The instructions may also contain warnings *e.g*. to keep a solution of adrenaline readily available in case of anaphylactic reaction following vaccination, *etc.*

### Methods of treatment, and administration of the vaccine

Compositions of the invention are suitable for administration to human patients, and can be used in a method of raising an immune response in a patient, comprising the step of adm inistering a composition of the invention to the patient.

These methods will generally be used to generate an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [190]. Antibody responses are typically measured by hemagglutination inhibition, by microneutralisation, by single radial immunodiffusion (SRID), and/or by single radial hemolysis (SRH). These assay techniques are well known in the art.

Compositions of the invention can be administered in various ways. The most preferred immunisation route is by intramuscular injection (*e.g*. into the arm or leg), but other available routes include subcutaneous injection, intranasal [191-193], oral [194], intradermal [195,196], transcutaneous, transdermal [197], *etc.*

Vaccines prepared according to the invention may be used to treat both children and adults. Influenza vaccines are currently recommended for use in pediatric and adult immunisation, from the age of 6 months. Thus the patient may be less than 1 year old, I-5 years old, 5-15 years old, 15-55 years old, or at least 55 years old. Preferred patients for receiving the vaccines are the elderly (*e.g*. ≥50 years old, ≥60 years old, and preferably ≥65 years), the young (*e.g.* ≤5 years old), hospitalised patients, healthcare workers, armed service and military personnel, pregnant women, the chronically ill, immunodeficient patients, patients who have taken an antiviral compound (*e.g*. an oseltamivir or zanamivir compound; see below) in the 7 days prior to receiving the vaccine, people with egg allergies and people travelling abroad. The vaccines are not suitable solely for these groups, however, and may be used more generally in a population.

Vaccines of the invention are particularly useful for treating patients in the 0-15 years old group.

Preferred compositions of the invention satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% scroprotection; (2) ≥30% seroconversion; and/or (3) a GMT increase of ≥2-fold. These criteria are based on open label studies with at least 50 patients.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g*. a parenteral prime and mucosal boost, a mucosal prime and parenteral boost, *etc.* Administration of more than one dose (typically two doses) is particularly useful in immunologically naive patients *e.g*. for people who have never received an influenza vaccine before, or for vaccines including a new HA subtype. Multiple doses will typically be administered at least 1 week apart (*e.g*. about 2 weeks, about 3 weeks, about 4 weeks, about 6 weeks, about 8 weeks, about 12 weeks, about 16 weeks, *etc*.).

Vaccines produced by the invention may be administered to patients at substantially the same time as (*e.g*. during the same medical consultation or visit to a healthcare professional or vaccination centre) other vaccines *e.g*. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the invention may be administered to patients at substantially the same time as (*e.g.* during the same medical consultation or visit to a healthcare professional) an antiviral compound, and in particular an antiviral compound active against influenza virus (*e.g*. oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof (*e.g*. the ethyl esters) and salts thereof (*e.g*. the phosphate salts). A preferred antiviral is (3R,4R,5S)-4-acetylamino-5-amino-3(1-ethylpropoxy)-1-cyclohexene-1-carboxylic acid, ethyl ester, phosphate (1:1), also known as oseltamivir phosphate (TAMIFLU™).

### General

The term "comprising" encompasses "including" as well as "consisting" *e.g*. a composition "comprising" X may consist exclusively of X or may include something additional *e.g*. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

Where a cell substrate is used for reassortment or reverse genetics procedures, or for viral growth, it is preferably one that has been approved for use in human vaccine production e.g. as in Ph Eur general chapter 5.2.3.

### BRIEF DESCRIPTION OF DRAWINGS

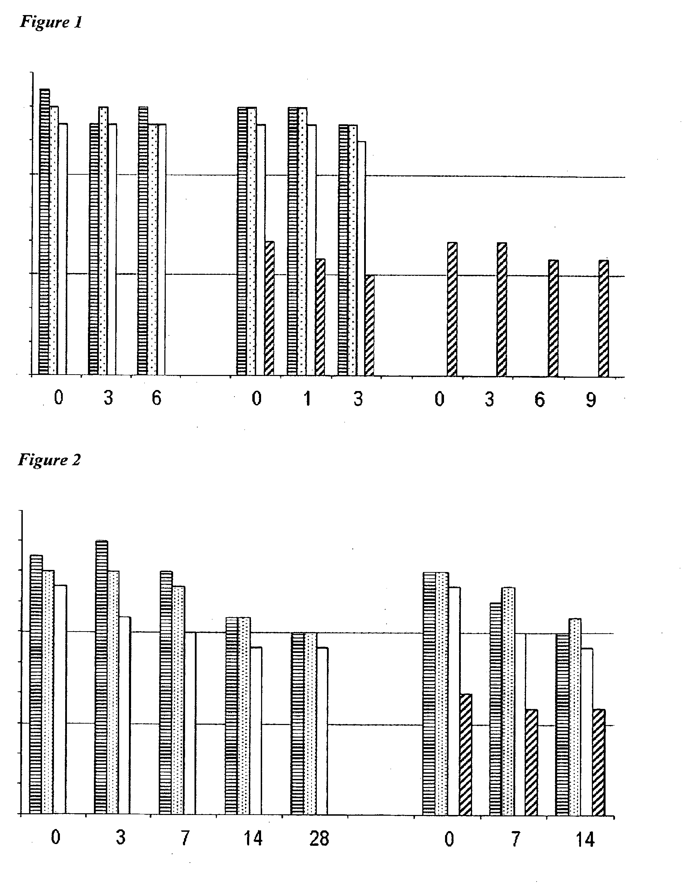

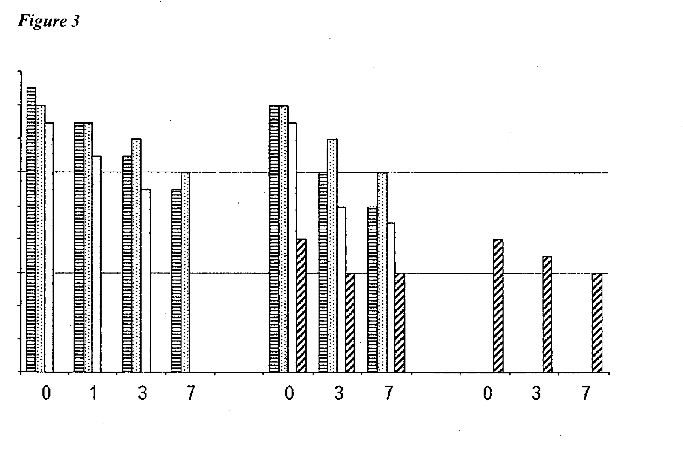

Figures 1 to 3 show vaccine stability during storage at (I) 2-8°C, (2) 23-27°C, or (3) 35-39°C. The figures on the X-axes show months in Figure 1 or days in Figures 2 & 3. The Y-axis shows HA levels, with the horizontal lines showing the stability thresholds for the seasonal antigens (upper) and the pandemic antigens (lower). In Figures 1 and 3 the three groups of bars are, from left to right: FLUAD™; 4-valent; and AFLUNOV™. In Figure 2 the two groups are FLUAD™ and 4-valent. At each timepoint data are shown, as relevant, for H1N (horizontal stripes), H3N2 (light shading), B (white) or H5N1 (cross-shading).

### MODES FOR CARRYING OUT THE INVENTION

### A-A-B-B vaccines

In recent years, Victoria/2/87-like and Yamagata/16/88-like lineages ('V' and 'Y' lineages) of influenza B virus have co-circulated, and their respective contributions to the overall seasonal epidemic illness burden has alternated from year to year. Vaccines in use over recent years have included either a V lineage or a Y lineage. From the 2001/02 season, the percentage of all US influenza cases that could potentially have been averted if the vaccine had included the other lineage has ranged from 0 to 29.9%, and in four of the five years starting with 2001/02 the proportion of influenza cases due to the mismatched B virus was actually greater than the proportion attributable to A/H1N1 virus:

| **Year** | **N** | **H3N2** | **H1N1** | **B all** | **B Y lineage** | **B V lineage** | **Missing coverage** |
|---|---|---|---|---|---|---|---|
| 2005 | 1019 | 55% | 13.2% | 31.5% | 5.8% | 25.6% | 25.6% |
| 2006 | | | | | (18.7%) | (81.3%) | |
| 2004 | 1075 | 65.9% | 1% | 33% | 24.5% | 8.5% | 8.5% |
| 2005 | | | | | (74.4%) | (25.6%) | |
| 2003 | 1024 | 92.6% | 0.3% | 6.9% | 6.4% | 0.4% | 6.4% |
| 2004 | | | | | (92.9%) | (7%) | |
| 2002 | 699 | 20.5% | 41.1% | 38.5% | 0.01% | 38.5% | - |
| 2003 | | | | | (0.1%) | (99.9%) | |
| 2001 | 690 | 54.0% | 4.3% | 38.7% | 8.8% | 29.9% | 29.9% |
| 2002 | | | | | (22.8%) | (77.1%) | |

| | | | | | | | |
|---|---|---|---|---|---|---|---|
| N is the number of influenza cases; percentages show the virus types responsible for those cases; the underlined figures for influenza B show the lineage that was in that season's vaccine; "Missing coverage" is the proportion of influenza cases that were caused by the influenza B virus lineage that was not in that season's vaccine. | | | | | | | |

Thus addition of the "missing" B virus lineage to form a 4-valent vaccine could have prevented up to 30% of influenza cases per season for the last 5 years, but egg-based manufacture techniques have prevented such vaccines from being produced. Cell culture techniques can overcome this problem.

### A-A-A-B vaccines (for reference)

Analysis of genomes of H3N2 influenza A virus strains sampled during 1999-2004 showed that, although the majority of viruses isolated after 2002 fell into a single phylogenetic group (clade A), multiple co-circulating viral lineages were present at particular time points [1]. In the 2003-04 influenza season a major drift variant emerged in both the northern and southern hemispheres, namely the A/Fujian/411/2002-like variant. Influenza in the USA in 2001/02 was predominantly H3N2-caused, and all antigenically characterized isolates matched the A/Moscow/10/1999 vaccine strain. In the next season, when disease was dominated by H1 and influenza B, a minority of antigenically-characterized H3N2 isolates were different from the Moscow/10/1999-like vaccine strain, probably coinciding with the emergence of the Fujian strain. The 2003/04 influenza season in the northern hemisphere was also dominated by the Fujian strain, but the vaccine strain being used contained the H3N2 from the previous year (A/Panama/2007/1999). This strain was a poor antigenic match to the Fujian strain, which led to reduced vaccine effectiveness. The Fujian strain had been rejected by the FDA because it had not originally been isolated in eggs [198,199] and no antigenically-similar egg-isolated strains were available. Eliminating the need for egg isolation and/or passaging would have prevented this situation, and cell culture techniques overcome this problem. Moreover, inclusion of two H3N2 strains in an influenza vaccine would avoid any problems caused by lack of cross-reactivity between A/Moscow/10/99-like and A/Fujian/411/2002-like strains.

### Vaccines including seasonal and pandemic strains (for reference)

Current seasonal influenza vaccines include hemagglutinin antigen from one H1N1 strain of influenza A virus, one H3N2 strain of influenza A virus, and one strain of influenza B virus. This seasonal combination has been supplemented by an antigen from a H5N1 strain of influenza virus i.e. a strain with the potential to cause an influenza pandemic.

A seasonal vaccine was prepared including purified surface glycoproteins from each of a A/New Caledonia H1N1 strain, a A/Wisconsin H3N2 strain and a B/Malaysia strain. The antigen dose was 30µg/ml, giving 15µg/strain/dose, but with a 15% overage. The bulk antigens were mixed at 2x strength and then diluted at a 1:1 volume ratio with MF59 oil-in-water emulsion adjuvant to give the final trivalent vaccine, which is sold under trade name FLUAD™.

A pre-pandemic vaccine was prepared based on purified surface glycoproteins from a H5N1 strain of influenza A virus. The antigen dose was 15µg/ml, giving 7.5µg/dose, but with a 15% overage. The bulk antigen at 2x strength was mixed at a 1:1 volume ratio with MF59 oil-in-water emulsion adjuvant to give the final vaccine (AFLUNOV™).

A 4-valent vaccine was prepared including the same four viral antigens. The seasonal antigens were included at the same HA concentrations as in FLUAD™ but the H5N1 antigen was included at 7.5µg/dose with no overage. The antigens were mixed at 2x strength and then diluted at a 1:1 ratio with MF59 oil-in-water emulsion adjuvant to give the final 4-valent vaccine. This vaccine is different from a simple mixture of FLUAD™ and AFLUNOV™ because the antigens are at their full dose in a 0.5ml volume, whereas a simple mixture would provide the full dose in a 1ml volume.

For stability studies vaccines were.all stored at: (1) refrigerated, 2-8°C, for at least 3 months; (2) at room temperature, 23-27°C, for at least 14 days; (3) at elevated temperature, 35-39°C. at least 7 days. Antigen levels were assayed at various timepoints during storage to check for stability. The composition is deemed unstable if antigen levels drop by more than 20% below the target level during the period of the study. Figures 1 to 3 show the results.
Figure 1 shows that the antigens in the FLUAD™ formulation were stable for up to 6 months when refrigerated, and that the addition of the H5N1 strain did not cause any of the seasonal antigens to drop below the stability threshold for the 3 months of the study. Similarly, the AFLUNOV™ formulation was stable for up to 9 months, and the addition of the seasonal antigens did not cause the H5N1 antigens to drop below the stability threshold for the 3 months of the study.
Figure 2 shows that the antigens in the FLUAD™ formulation were stable for 7 days at room temperature, but that the influenza B antigen dropped below the stability threshold at 14 days. The antigens were also stable for 7 days in the presence of the H5N1 strain. The H5N1 antigen remained stable in the 4-valent composition for at least 14 days.
Figure 3 shows that the antigens in the FLUAD™ formulation were stable for I day at elevated temperature, but that the influenza B antigen dropped below the stability threshold at 3 days. The AFLUNOV™ formulation was stable for 7 days. The H5N1 antigen remained stable in the 4-valent composition for 7 days and it did not cause the seasonal antigens to drop below the threshold any more quickly than in the FLUAD™ formulation.

Thus a H5N1 antigen can be combined with seasonal antigens, in the presence of an adjuvant, without reducing stability of any of the 4 antigens.

In separate efficacy studies the AGRIPPAL™ seasonal vaccine and the adjuvanted AFLUNOV™ H5N1 vaccine are administered to human patients individually, concomitantly, or are mixed and then administered as a combination vaccine. Patients are split into 8 groups of 50 patients each. Groups 1 to 3 receive concomitant vaccines at time zero. Groups 4 to 6 receive the combination vaccine at time zero. Group 7 receives AFLUNOV™ at time zero. Group 8 receives AGRIPPAL™ at time zero.

At day 21, groups 1 and 4 receive no extra vaccine, but all other groups receive a second vaccine. Groups 2 and 5 receive the combination vaccine. Groups 3, 6 and 8 receive AFLUNOV™. Group 7 receives AGRIPPAL™.

At day 365 all groups receive AFLUNOV™ and immune responses are assessed.

It will be understood that the invention has been described by way of example only and modifications may be made whilst remaining within the scope of the invention.

### REFERENCES

[1] Holmes et al. (2005) PLoS Biol. 3(9):e300.
[2] World Health Organisation (2005) Emerging Infections Diseases 11(10) : 1515-21.
[3] Rota et al. (1992) JGen Virol 73:2737-42.
[4] GenBank sequence GI:325176.
[5] McCullers et al. (1999) J Virol 73:7343-8.
[6] GenBank sequence GI:325237.
[7] Hoffmann et al. (2002) Vaccine 20:3165-3170.
[8] Subbarao et al. (2003) Virology 305:192-200.
[9] Liu et al. (2003) Virology 314:580-590.
[10] Ozaki et al. (2004) J. Virol. 78:1851-1857.
[11] Webby et al. (2004) Lancet 363:1099-1103.
[12] WO00/60050.
[13] WO01/04333.
[14] US patent 6649372.
[15] Neumann et al. (2005) Proc Natl Acad Sci USA 102:16825-9.
[16] WO2006/067211.
[17] WO01/83794.
[18] Hoffmann et al. (2000) Virology 267(2):310-7.
[19] Herlocher et al. (2004) J Infect Dis 190(9):1627-30.
[20] Le et al. (2005) Nature 437(7062):1108.
[21] Gambaryan & Matrosovich (1992) J Virol Methods 39(1-2):111-23.
[22] Mastrosovich et al. (1999) J Virol 73: 1146-55.
[23] Stevens et al. (2006) J Mol Biol 355:1143-55.
[24] Couceiro & Baum (1994) Mem Inst Oswaldo Cruz 89(4):587-91.
[25] WO02/28422.
[26] WO02/067983.
[27] WO02/074336.
[28] WO01/21151.
[29] WO02/097072.
[30] WO2005/113756.
[31] Huckriede et al. (2003) Methods Enzymol 373:74-91.
[32] Treanor et al. (1996) J Infect Dis 173:1467-70.
[33] Keitel et al. (1996) Clin Diagn Lab Immunol 3:507-10.
[34] Saito et al. (2004) J Med Virol 74(2):336-43.
[35] Kistner et al. (1998) Vaccine 16:960-8*.*
[36] Kistner et al. (1999) Dev Biol Stand 98:101-110.
[37] Bruhl et al. (2000) Vaccine 19:1149-58.
[38] Pau et al. (2001) Vaccine 19:2716-21.
[39] *hitp:*//*www.atcc.org*/
*[40] http:*//*locus.umdnj.edu*/
[41] WO03/076601.
[42] W02005/042728.
[43] WO03/043415.
[44] WO01/85938.
[45] WO2006/108846.
[46] WO97/37000.
[47] Brands et al. (1999) Dev Biol Stand 98:93-100.
[48] Halperin et al. (2002) Vaccine 20:1240-7.
[49] Tree et al. (2001) Vaccine 19:3444-50.
[50] EP-A-1260581 (WO01/64846).
[51] WO2006/071563.
[52] WO2005/113758.
[53] WO97/37001.
[54] W02006/027698.
[55] EP-B-0870508.
[56] US 5948410.
[57] Lundblad (2001) Biotechnology and Applied Biochemistry 34:195-197.
[58] Guidance for Industry: Bioanalytical Method Validation. U.S. Department of Health and Human Services Food and Drug Administration Center for Drug Evaluation and Research (CDER) Center for Veterinary Medicine (CVM). May 2001.
[59] Ji et al. (2002) Biotechniques. 32:1162-7.
[60] Briggs (1991) J Parenter Sci Technol. 45:7-12.
[61] Lahijani et al. (1998) Hum Gene Ther. 9:1173-80.
[62] Lokteff et al. (2001) Biologicals. 29:123-32.
[63] Gennaro (2000) Remington: The Science and Practice of Pharmacy. 20th edition, ISBN: 0683306472.
[64] Banzhoff (2000) Immunology Letters 71:91-96.
[65] Nony et al. (2001) Vaccine 27:3645-51.
[66] US patent 6,372,223.
[67] WO00/15251.
[68] WO01/22992.
[69] Hehme et al. (2004) Virus Res. 103(1-2):163-71.
[70] US patent 6355271.
[71] WO00/23105.
[72] US 5,057,540.
[73] WO96/33739.
[74] EP-A-0109942.
[75] WO96/11711.
[76] WO00/07621.
[77] Barr et al. (1998) Advanced Drug Delivery Reviews 32:247-271.
[78] Sjolanderet et al. (1998) Advanced Drug Delivery Reviews 32:321-338.
[79] Pizza et al. (2000) Int J Med Microbiol 290:455-461.
[80] WO95/17211.
[81] WO98/42375.
[82] Singh et al] (2001) J Cont Release 70:267-276.
[83] WO99/27960.
[84] US 6,090,406
[85] US 5,916,588
[86] EP-A-0626169.
[87] WO99/52549.
[88] WO01/21207.
[89] WO01/21152.
[90] WO02/072012.
[91] Signorelli & Hadden (2003) Int Immunopharmacol 3(8):1177-86.
[92] WO2004/064715.
[93] Cooper (1995) Pharm Biotechnol 6:559-80.
[94] WO90/14837.
[95] Podda & Del Giudice (2003) Expert Rev Vaccines 2:197-203.
[96] Podda (2001) Vaccine 19: 2673-2680.
[97] Vaccine Design: The Subunit and Adjuvant Approach (eds. Powell & Newman) Plenum Press 1995 (ISBN 0-306-44867-X).
[98] Vaccine Adjuvants: Preparation Methods and Research Protocols (Volume 42 of Methods in Molecular Medicine series). ISBN: 1-59259-083-7. Ed.O'Hagan.
[99] Allison & Byars (1992) Res Immunol 143:519-25.
[100] Hariharan et al. (1995) Cancer Res 55:3486-9.
[101] US-2007/014805.
[102] Suli et al. (2004) Vaccine 22(25-26):3464-9.
[103] WO95/11700.
[104] US patent 6,080,725.
[105] WO2005/097181.
[106] WO2006/113373.
[107] Han et al. (2005) Impact of Vitamin E on Immune Function and Infectious Diseases in the Aged at Nutrition, Immunefunclions and Health EuroConference, Paris, 9-10 June 2005.
[108] US- 6630161.
[109] Hayden et al. (1998) J Clin Invest 101 (3):643-9.
[110] Tassignon et al. (2005) J Immunol Meth 305:188-98.
[111] Myers et al. (1990) pages 145-156 of Cellular and molecular aspects of endotoxin reactions*.*
[112] Ulrich (2000) Chapter 16 (pages 273-282) of reference 98.
[113] Johnson et al. (1999) J Med Chem 42:4640-9.
[114] Baldrick et al. (2002) Regulatory Toxicol Pharmacol 35:398-413.
[115] US 4,680,338.
[116] US 4,988,815.
[117] WO92/15582.
[118] Stanley (2002) Clin Exp Dermatol 27:571-577.
[119] Wu et al. (2004) Antiviral Res. 64(2):79-83.
[120] Vasilakos et al. (2000) Cell Immunol. 204(1):64-74.
[121] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[122] Jones (2003) Curr Opin Investig Drugs 4:214-218.
[123] WO2004/060308.
[124] WO2004/064759.
[125] US 6,924,271.
[126] US2005/0070556.
[127] US 5,658,731.
[128] US patent 5,011,828. ,
[129] W02004/87153.
[130] US 6,605,617.
[131] WO02/18383.
[132] W02004/018455.
[133] WO03/082272.
[134] Dyakonova et al. (2004) Int Immunopharmacol 4(13):1615-23.
[135] FR-2859633.
[136] WO2006/002422.
[137] Johnson et al. (1999) Bioorg Med Chem Lett 9:2273-2278.
[138] Evans et al. (2003) Expert Rev Vaccines 2:219-229.
[139] Andrianov et al. (1998) Biomaterials 19:109-115.
[140] Payne et al. (1998) Adv Drug Delivery Review 31:185-196.
[141] De Libero et al, Nature Reviews Immunology, 2005, 5: 485-496
[142] US patent 5,936,076.
[143] Oki et al, J. Clin. Investig., 113: 1631-1640
[144] US2005/0192248
[145] Yang et al, Angew. Chem. Int. Ed., 2004, 43: 3818-3822
[146] WO2005/102049
[147] Goff et al, J. Am. Chem., Soc., 2004, 126: 13602-13603
[148] WO03/105769
[149] Thompson et al. (2003) Methods in Molecular Medicine 94:255-266.
[150] Kandimalla et al. (2003) Nucleic Acids Research 31:2393-2400.
[151] WO02/26757.
[152] WO99/62923.
[153] Krieg (2003) Nature Medicine 9:831-835.
[154] McCluskie et al. (2002) FEMS Immunology and Medical Microbiology 32:179-185.
[155] WO98/40100.
[156] US patent 6,207,646.
[157] US patent 6,239,116.
[158] US patent 6,429,199.
[159] Kandimalla et al. (2003) Biochemical Society Transactions 31 (part 3):654-658.
[160] Blackwell et al. (2003) J Immunol 170:40614068.
[161] Krieg (2002) Trends Immunol 23:64-65.
[162] WO01/95935.
[163] Kandimalla et al. (2003) BBRC 306:948-953.
[164] Bhagat et al. (2003) BBRC 300:853-861.
[165] WO03/035836.
[166] WO01/22972.
[167] Schellack et al. (2006) Vaccine 24:5461-72.
[168] Thompson et al. (2005) J Leukoc Biol 78: 'The low-toxicity versions of LPS, MPL® adjuvant and RC529, are efficient adjuvants for CD4+ T cells'.
[169] UK patent application GB-A-2220211.
[170] WO 94/21292.
[171] WO94/00153.
[172] WO95/17210.
[173] WO96/26741.
[174] WO93/19780.
[175] WO03/011223.
[176] Meraldi et al. (2003) Vaccine 21:2485-2491.
[177] Pajak et al. (2003) Vaccine 21:836-842.
[178] US-6586409.
[179] Wong et al. (2003) J Clin Pharmacol 43(7):735-42.
[180] US2005/0215517.
[181] Vaccine Design: The Subunit and Adjarvant Approach (eds. Powell & Newman) Plenum Press 1995 (ISBN 0-306-44867-X).
[182] WO2005/089837.
[183] US patent 6,692,468.
[184] WO00/07647.
[185] WO99/17820.
[186] US patent 5,971,953.
[187] US patent 4,060,082.
[188] EP-A-0520618.
[189] WO98/01174.
[190] Potter & Oxford (1979) Br Med Bull 35: 69-75.
[191] Greenbaum et al. (2004) Vaccine 22:2566-77.
[192] Zurbriggen et al. (2003) Expert Rev Vaccines 2:295-304.
[193] Piascik (2003) J Am Pharm Assoc (Wash DC). 43:728-30.
[194] Mann et al. (2004) Vaccine 22:2425-9.
[195] Halperin et al. (1979) Am J Public Health 69:1247-50.
[196] Herbert et al. (1979) J Infect Dis 140:234-8.
[197] Chen et al. (2003) Vaccine 21:2830-6.
[198] Palese (2006) Emerging Infectious Diseases 12:61-65.
[199] WHO (2003) Weekly epidemiological record 78:73-80

## Claims

1. A vaccine comprising hemagglutinin from: (i) a H1N1 strain; (ii) a H3N2 strain; (iii) a B/Victoria/2/87-like strain; and (iv) a B/Yamagata/16/88-like strain, wherein the viruses were grown in cell culture and the strains have not been passaged through eggs at any stage between isolation from a patient and replication in cell culture, and wherein the vaccine further comprises an adjuvant comprising an oil-in-water emulsion.

2. The vaccine of claim 1, wherein the antigens are whole virion, split virion, or purified surface antigen.

3. The vaccine of claim 1 or claim 2, wherein the strains were grown in MDCK cells.

4. The vaccine of any of claims 1-3, wherein the oil-in-water emulsion comprises sub-micron droplets.

5. The vaccine of claim 4, wherein the oil comprises squalene.

6. The vaccine of any preceding claim, containing substantially the same mass of hemagglutinin for each of the four influenza virus strains.

7. A kit for preparing the vaccine of any preceding claim comprising: (i) a first container containing an influenza vaccine comprising hemagglutinin from a H1N1 strain, a H3N2 strain, and a first influenza B virus strain; and (ii) a second container containing an influenza vaccine comprising hemagglutinin from a second influenza B virus strain, wherein one of the first and the second influenza B virus strains is a B/Victoria/2/87-like strain and the other is a B/Yamagata/16/88-like strain.

8. A kit for preparing the vaccine of any of claims 1-6 comprising (i) a first component comprising the influenza virus antigens, in a first container; and (ii) a second component comprising an oil-in-water emulsion, in a second container.

9. Antigens from four different strains of influenza virus comprising hemagglutinin for use in raising an immune response in a patient, wherein the four strains include: (i) a H1N1 strain; (ii) a H3N2 strain; (iii) a B/Victoria/2/87-like strain; and (iv) a B/Yamagata/16/88-like strain, wherein the viruses were grown in cell culture and the strains have not been passaged through eggs at any stage between isolation from a patient and replication in cell culture, and the antigens are adjuvanted with an oil-in-water emulsion.

10. A method for preparing a vaccine comprising: (i) a H1N1 strain; (ii) a H3N2 strain; (iii) a B/Victoria/2/87-like strain; and (iv) a B/Yamagata/16/88-like strain, wherein the method comprises the steps of:
a) growing four different strains of influenza virus in cell culture;
b) preparing an antigen composition from each of the viruses grown in step (a), wherein the antigen composition includes hemagglutinin; and
c) combining the antigen compositions with a pharmaceutical carrier, to give the vaccine; wherein the strains have not been passaged through eggs at any stage between isolation from a patient and replication in cell culture, and the vaccine further comprises an adjuvant comprising an oil-in-water emulsion.

## Patentansprüche

1. Impfstoff, umfassend Hämagglutinin aus: (i) einem H1N1-Stamm, (ii) einem H3N2-Stamm, (iii) einem B/Victoria/2/87-ähnlichen Stamm und (iv) einem B/Yamagata/16/88-ähnlichen Stamm, wobei die Viren in Zellkultur herangezogen wurden und die Stämme in keinem Stadium zwischen der Isolierung aus einem Patienten und der Replikation in Zellkultur durch Eier passagiert wurden, und wobei der Impfstoff weiterhin ein eine Öl-in-Wasser-Emulsion umfassendes Adjuvans umfasst.

2. Impfstoff nach Anspruch 1, wobei die Antigene Ganzviren, Spaltviren oder aufgereinigte Oberflächenantigene sind.

3. Impfstoff nach Anspruch 1 oder 2, wobei die Stämme in MDCK-Zellen herangezogen wurden.

4. Impfstoff nach einem der Ansprüche 1-3, wobei die Öl-in-Wasser-Emulsion Submikrontröpfchen umfasst.

5. Impfstoff nach Anspruch 4, wobei das Öl Squalen umfassst.

6. Impfstoff nach einem der vorhergehenden Ansprüche, welcher für jeden der vier Influenza-Virusstämme im Wesentlichen die gleiche Masse an Hämagglutinin umfasst.

7. Kit zur Herstellung des Impfstoffs nach einem der vorhergehenden Ansprüche, umfassend: (i) ein erstes Behältnis, welches einen Hämagglutinin aus einem H1N1-Stamm, einem H3N2-Stamm und einem ersten Influenza-B-Virusstamm umfassenden Influenza-Impfstoff enthält, und (ii) ein zweites Behältnis, welches einen Hämagglutinin aus einem zweiten Influenza-B-Virusstamm umfassenden Influenza-Impfstoff enthält, wobei es sich bei einem der ersten und zweiten Influenza-B-Virusstämme um einen B/Victoria/2/87-ähnlichen Stamm und bei dem anderen um einen B/Yamagata/16/88-ähnlichen Stamm handelt.

8. Kit zur Herstellung des Impfstoffs nach einem der Ansprüche 1-6, umfassend (i) eine erste Komponente, welche die Influenza-Virus-Antigene umfasst, in einem ersten Behältnis, und (ii) eine zweite Komponente, welche eine Öl-in-Wasser-Emulsion umfasst, in einem zweiten Behältnis.

9. Hämagglutinin umfassende Antigene aus vier verschiedenen Influenza-Virusstämmen zur Verwendung beim Hervorrufen einer Immunantwort in einem Patienten, wobei die vier Stämme folgende einschließen: (i) einen H1N1-Stamm, (ii) einen H3N2-Stamm, (iii) einen B/Victoria/2/87-ähnlichen Stamm und (iv) einen B/Yamagata/16/88-ähnlichen Stamm, wobei die Viren in Zellkultur herangezogen wurden und die Stämme in keinem Stadium zwischen der Isolierung aus einem Patienten und der Replikation in Zellkultur durch Eier passagiert wurden, und die Antigene mit einer Öl-in-Wasser-Emulsion ergänzt werden.

10. Verfahren zum Herstellen eines Impfstoffes, welcher (i) einen H1N1-Stamm, (ii) einen H3N2-Stamm, (iii) einen B/Victoria/2/87-ähnlichen Stamm und (iv) einen B/Yamagata/16/88-ähnlichen Stamm umfasst, wobei das Verfahren die folgenden Schritte umfasst:
a) Heranziehen von vier verschiedenen Influenza-Virusstämmen in Zellkultur,
b) Herstellen einer Antigenzusammensetzung von jedem der in Schritt (a) herangezogenen Viren, wobei die Antigenzusammensetzung Hämagglutinin einschließt, und
c) Kombinieren der Antigenzusammensetzungen mit einem pharmazeutischen Träger unter Erhalt des Impfstoffs, wobei die Stämme in keinem Stadium zwischen der Isolierung aus einem Patienten und der Replikation in Zellkultur durch Eier passagiert wurden und der Impfstoff weiterhin ein eine Öl-in-Wasser-Emulsion umfassendes Adjuvanz umfasst.

## Revendications

1. Vaccin comprenant une hémagglutinine provenant de : (i) une souche H1N1; (ii) une souche H3N2 ; (iii) une souche du type B/Victoria/2/87 ; et (iv) une souche du type B/Yamagata/16/88, dans lequel les virus ont été cultivés dans une culture cellulaire et les souches n'ont pas été passées sur des oeufs à un quelconque stade entre le moment de l'isolement, à partir d'un patient, et la réplication en culture cellulaire, et dans lequel le vaccin comprend en outre un adjuvant, qui comprend une émulsion huile-dans-eau.

2. Vaccin selon la revendication 1, dans lequel les antigènes sont un virion entier, un virion fragmenté ou un antigène de surface purifié.

3. Vaccin selon la revendication 1 ou la revendication 2, dans lequel les souches ont été cultivées dans des cellules MDCK.

4. Vaccin selon l'une quelconque des revendications 1 à 3, dans lequel l'émulsion huile-dans-eau comprend des gouttelettes dont la taille est inférieure au micron.

5. Vaccin selon la revendication 4, dans lequel l'huile comprend du squalène.

6. Vaccin, selon l'une quelconque des revendications précédentes, contenant substantiellement la même masse d'hémagglutinine pour chacune des quatre souches de virus de la grippe.

7. Trousse destinée à préparer le vaccin selon l'une quelconque des revendications précédentes, comprenant : (i) un premier conteneur contenant un vaccin contre la grippe, qui comprend une hémagglutinine provenant d'une souche de H1N1, d'une souche de H3N2 et d'une souche du virus de la grippe B ; et (ii) un deuxième conteneur contenant un vaccin contre la grippe, qui comprend une hémagglutinine provenant d'une deuxième souche de virus de la grippe B, dans laquelle l'une des souches de virus de la grippe B parmi la première et la deuxième est une souche du type B/Victoria/2/87 et l'autre est une souche du type B/Yamagata/16/88.

8. Trousse destinée à préparer le vaccin selon l'une quelconque des revendications 1 à 6, comprenant : (i) un premier composant comprenant les antigènes de virus de la grippe, dans un premier conteneur ; et (ii) un deuxième composant comprenant une émulsion huile-dans-eau, dans un deuxième conteneur.

9. Antigènes provenant de quatre souches différentes de virus de la grippe, comprenant de l'hémagglutinine, destinés à être utilisés dans la génération d'une réponse immunitaire chez un patient, dans laquelle les quatre souches comprennent : (i) une souche H1N1; (ii) une souche H3N2 ; (iii) une souche du type B/Victoria/2/87 ; et (iv) une souche du type B/Yamagata/16/88, dans laquelle les virus ont été cultivés dans une culture cellulaire et les souches n'ont pas été passées sur des oeufs à un quelconque stade entre le moment de l'isolement, à partir d'un patient, et la réplication en culture cellulaire, et les antigènes sont adjuvantés avec une émulsion huile-dans-eau.

10. Procédé de préparation d'un vaccin comprenant : (i) une souche H1N1; (ii) une souche H3N2 ; (iii) une souche du type B/Victoria/2/87 ; et (iv) une souche du type B/Yamagata/16/88, dans laquelle le procédé comprend les étapes consistant à :
a) cultiver quatre souches différentes de virus de la grippe dans une culture cellulaire ;
b) préparer une composition antigénique provenant de chacun des virus cultivés dans l'étape (a), dans laquelle la composition antigénique comprend de l'hémagglutinine ; et
c) combiner les compositions antigéniques avec un véhicule pharmaceutique, pour donner le vaccin ; dans lequel les souches n'ont pas été passées sur des oeufs à un quelconque stade entre le moment de l'isolement, à partir d'un patient, et la réplication en culture cellulaire, et le vaccin comprend en outre un adjuvant, qui comprend une émulsion huile-dans-eau.
